# EUROPEAN PATENT SPECIFICATION

(11) **EP 3 897 857 B1**
(45) Date of publication and mention of the grant of the patent: **02.07.2025**
(21) Application number: 18852734.5
(22) Date of filing: 19.12.2018
(51) Int. Cl.: A61Q 1/00, A61K 8/73, A61Q 19/00, A61K 8/02, A61K 8/03, A61K 8/04

(54) **REDISPERSIBLE TWO-LAYER COSMETIC**
REDISPERGIERBARES AUS ZWEI SCHICHTEN BESTEHENDES KOSMETISCHES PRODUKT
PRODUIT COSMÉTIQUE BICOUCHE REDISPERSIBLE

(43) Date of publication of application: 27.10.2021
(73) Proprietor: L V M H RECHERCHE, 45800 St. Jean de Braye (FR)
(72) Inventor: KITAMURA, Miyako, Tokyo 102--0092 (JP); OZAWA, Mai, Tokyo 102--0092 (JP); SAKODA, Takayoshi, Tokyo 102--0092 (JP)
(74) Representative: Regimbeau
(86) International application number: PCT/IB2018/001593
(87) International publication number: WO 2020/128555

(56) References cited:
- WO-A1-2013/042069
- WO-A1-2016/204836
- WO-A1-2019/002579
- WO-A1-2019/002579
- WO-A1-2020/128558
- FR-A1- 3 008 312
- JP-A- 2001 097 841
- JP-A- 2002 255 739
- JP-A- 2007 269 674
- JP-K1- 2001 097 841
- JP-K1- 2002 255 739
- MARGUERITE RINAUDO: "Gelation of Polysaccharides", JOURNAL OF INTELLIGENT MATERIAL SYSTEMS AND STRUCTURES., vol. 4, no. 2, 27 April 1993 (1993-04-27), US, pages 210 - 215, XP055604900, ISSN: 1045-389X, DOI: 10.1177/1045389X9300400210
- RYAN LOFTUS: "Calcium Alginate Microbead Production via an Air Assisted Shearing Process", 1 January 2016 (2016-01-01), XP055605006, Retrieved from the Internet <URL:https://pdfs.semanticscholar.org/f03a/762d2ea3aa752514d2d709e4e13e6b83f10a.pdf> [retrieved on 20190915]
- KATHIRVEL GANESAN ET AL: "Review on the Production of Polysaccharide Aerogel Particles", MATERIALS, vol. 11, no. 11, 31 October 2018 (2018-10-31), pages 2144, XP055556734, DOI: 10.3390/ma11112144
- B.MILOW ET AL.: "Report on the possible production processes of organic and hybrid aerogels and comparison with the state of the art", NANOHYBRIDS, April 2017 (2017-04-01), pages 1 - 125, XP002792948, Retrieved from the Internet <URL:http://nanohybrids.eu/wp-content/uploads/2017/04/D1_3_Report-on-the-possible-production-processes-of-organic-and-hybrid-aerogels-and-comparison-with-the-state-of-the-art_TUHH_Final.pdf> [retrieved on 20190712]

## Description

### Technical Field

The present invention relates to a redispersible two-layer cosmetic.

### Background Art

Multilayer cosmetics are composed of multiple layers (for example, a powder layer and an aqueous layer, or an aqueous layer and an oil layer). Many different technologies for multilayer cosmetics continue to be developed because such multilayer cosmetics provide excellent aesthetic appearance and are very attractive for consumers.

Japanese Unexamined Patent Publication No. 2013-177366 (equivalent EP2810639B1) , for example, discloses a redispersible powder-dispersed cosmetic comprising (A) succinic acid and/or its salt, (B) bentonite and (C) a hydrophilic surfactant. But in a two-layer cosmetic composed of an aqueous layer and a powder layer comprising specific powders such as bentonite, cellulose and a synthetic polymer, it is often the case that the components become adsorbed onto the skin due to the moistness of the powder layer, impacting the long-lasting moisturizing effect searched by the consumers.

Also, Japanese Unexamined Patent Publication No. 2007-126394 discloses a multilayer cosmetic comprising a copolymer obtained by polymerizing a specific polyethylene oxide macromonomer, a hydrophobic monomer and a crosslinkable monomer, with 5 to 40 mass% of a liquid oil, and with the surfactant content being within a specified range. Japanese Unexamined Patent Publication No. 2014-208634 discloses a multilayer cosmetic comprising the following components (a) to (d): (a) a nonvolatile hydrocarbon oil having a viscosity of 30 to 400 mm²/s at 25°C, (b) a silicone oil having a viscosity of 30 to 400 mm²/s at 25°C, (c) water and (d) an ester of isostearic acid and polyglycerin.

But such a two-layer cosmetic composed of an aqueous layer and an oil layer tends to produce an oily sticky feel, impacting the smoothness searched by the consumers. JP 2002 255739 A and JP 2001 097841 A (NIKKO SEIYAKU KK) disclose a bi-layered cosmetic having re-eulsification property when it is shaken, having bilayer separation property when it stands at rest and provides soothing and moisturizing effect. JP 2007 269674 A (KOSE CORP) discloses a bilayer type makeup remover.

So there is still a need for providing a two-layer cosmetic that is improved in terms of both long-lasting moisturizing effect and smoothness.

Research carried out by the present inventors has led to the discovery of a novel redispersible two-layer cosmetic composed of specific gel particles and an aqueous phase, and the two-layer cosmetic has been demonstrated to exhibit improved long-lasting moisturizing effect and smoothness.

### Summary of the Invention

The invention relates to a redispersible two-layer cosmetic comprising an aqueous phase and polysaccharide gel particles having a mean particle size of 0.1 to 1000 µm, wherein the aqueous phase comprises a polyol and/or a mono-alcohol, and wherein the redispersible two-layer cosmetic consists of a bottom layer formed by a plurality of gel particles aggregates, and an upper layer composed of the aqueous phase over the bottom layer, wherein the mean particle size is measured using a Laser scattering particle size distribution analyzer.
The expression 'redispersible two-layer cosmetic' according to the invention, means that the composition provides a good redispersibility of polysaccharide gel particles (bottom layer) in the aqueous phase (upper layer) when shaken for use while being able to give a clear supernatant when not-in-use. So, settling and dispersion of the gel particle in the aqueous phase takes place repeatedly through cycles of standing at rest (not-in-use) and agitation (shaken).
The term 'two-layer' cosmetic according to the invention, means that the composition presents two visually distinct layers, when it stands at rest (not-in-use), ie an upper layer comprising the aqueous phase and a bottom layer comprising the polysaccharide gel particles.
The redispersible two-layer cosmetic of the invention exhibits improved long-lasting moisturizing effect and smoothness.

The polysaccharide may be agar. In this case, as a gel particle, a pulverized form of an agar gel may be used and the agar gel is obtained by gelating the agar swelled with a gel-forming aqueous phase (second layer).

The viscosity of the two-layer cosmetic may be 0,001 Pa.s to 0,2 Pa.s at 25°C.

The aqueous phase comprises a polyol or a mono-alcohol. The aqueous phase may comprise an aryloxyalkanol such as phenoxyethanol.

### Advantageous Effects of Invention

With the invention it is possible to provide a redispersible two-layer cosmetic that is improved in terms of both long-lasting moisturizing effect and smoothness.

### Detailed Description of the Invention

Embodiments of the invention will now be described. However, the present invention is not limited to the embodiments described below.

The redispersible two-layer cosmetic of this embodiment contains an aqueous phase (first layer, ie upper layer), and polysaccharide gel particles having a mean particle size of 0.1 to 1000 µm (second layer, ie bottom layer). As used herein, the term "two-layer cosmetic" refers to a cosmetic having two visually distinct layers when not-in-use, i.e a plurality of gel particles separate from the aqueous phase.

When the two-layer cosmetic of this embodiment undergoes a cycle of standing at rest (not-in-use) and agitation (shaken), settling and dispersion of the gel particles takes place in the aqueous phase. Specifically, during settling (for example, when approximately 100 mL is housed in a container with a capacity of 120 mL and allowed to stand for one day or longer), the gel particles settle in the aqueous phase, so that the two-layer cosmetic consists of a bottom layer formed by a plurality of gel particles aggregates, and an upper layer composed of the aqueous phase over the bottom layer. The gel particles are composed of components such that, under usual storage temperature (for example, room temperature, or 5 to 30°C) and storage time (for example, 3 years) conditions for a two-layer cosmetic, they do not elute into the aqueous phase and lose their form, and the particles do not adhere together and form aggregates (masses). The aqueous phase may also be present between the gel particles. When agitated (shaken), however, the gel particles become dispersed in the aqueous phase, and therefore the two-layer cosmetic comprises the aqueous phase and the gel particles dispersed in the aqueous phase.

### Aqueous phase (first layer)

The aqueous phase comprises water-soluble components in water. Water-soluble components are components that dissolve to at least 0.1 g per 100 g of water.

Water-soluble components comprise polyols, mono-alcohols (excluding those qualifying as preservatives), preservatives, perfumes, solubilizers, salting-out agents, pH regulators, surfactants, whitening agents, anti-inflammatory agents, colorants, and non-gelating polysaccharides (for example, xanthan gum, sodium hyaluronate, gum arabic, xanthan gum, guar gum and succinoglycan).

The valency of a polyol may be 2 to 4, or 2 to 3, for example. In other words, the polyol may comprise diols and/or triols. Examples of polyols comprise diols such as butylene glycol (1,3-butylene glycol), pentylene glycol, propanediol, dipropylene glycol, polyalkylene glycols (such as polyethylene glycol), and triols such as glycerin. These polyols may be used alone or in combinations of two or more.

When the aqueous phase comprises or consists of a polyol, the total polyol content may be 1 to 30 mass%, 5 to 20 mass% or 7 to 15 mass%, based on the total mass of the two-layer cosmetic. The term 'mass%' is also referred as % by weight.

Mono-alcohols (C1-C5) comprise ethanol, propanol, isopropanol and butanol. In a particular embodiment, the mono-alcohol is ethanol. These mono-alcohols may also be used alone or in combinations of two or more. When the aqueous phase comprises or consists of a mono-alcohol, the total mono-alcohol content may be 1 to 20 mass%, 2 to 15 mass% or 3 to 10 mass% based on the total mass of the two-layer cosmetic.

Preservatives comprise aryloxyalkanols such as phenoxyethanol, and ethylenediaminetetraacetic acid (EDTA) disodium, and ethylenediaminetetraacetic acid (EDTA) tetrasodium. The aqueous phase may comprise an aryloxyalkanol as the preservative, but it preferably comprises phenoxyethanol. These preservatives may also be used alone or in combinations of two or more. When the aqueous phase comprises a preservative, the preservative content may be 0.01 to 5 mass%, 0.01 to 2 mass%, 0.1 to 2 mass%, 0.5 to 2 mass% or 0.7 to 2 mass%, based on the total mass of the two-layer cosmetic.

Solubilizers comprise PPG-6 decyl tetradeceth-20, PPG-6 decyl tetradeceth-30, PEG-60 hydrogenated castor oil, PEG-80 hydrogenated castor oil, PEG-60 glyceryl isostearate, PEG-20 sorbitan cocoate and PEG-20 sorbitan isostearate. When the aqueous phase comprises a solubilizer, the total solubilizer content may be 0.01 to 5 mass%, 0.05 to 1 mass% or 0.05 to 0.5 mass%, based on the total mass of the two-layer cosmetic.

Salting-out agents comprise sodium chloride, potassium chloride, calcium chloride, sodium sulfate and magnesium sulfate. When the aqueous phase comprises a salting-out agent, the total salting-out agent content may be 0.1 to 10 mass%, 0.5 to 5 mass% or 0.7 to 2 mass%, based on the total mass of the two-layer cosmetic.

The aqueous phase content may be 85.0 to 99.9 mass% or 90.0 to 99.5 mass%, based on the total mass of the two-layer cosmetic.

### Polysaccharide gel particles (second layer)

The gel particles comprise a gellable polysaccharide and a solvent in the second layer for formation of the gel (a gel-forming aqueous phase of the second layer). Examples of gellable polysaccharides (hereunder also referred to simply as "polysaccharides") comprise agar, carrageenan (such as kappa-carrageenan and iota-carrageenan), gellan gum, sodium alginate, tamarind gum, mannan and locust bean gum, and mixtures thereof. These polysaccharides may likewise be used alone or in combinations of two or more. The polysaccharide is preferably at least one selected from the group consisting of agar, carrageenan and gellan gum, and more preferably agar. The gel-forming aqueous phase may employ any of the components mentioned above for the aqueous phase of the first layer. The components used in the gel-forming aqueous phase may be the same as in the aqueous phase described above (first layer), or different ones. In a particular embodiment, the solvent used for formation of the polysaccharide gel is water.

Gel particles can be obtained by the following method, as an example. First, the gellable polysaccharide is swelled with the aqueous phase (second layer) by heating if necessary. The temperature for swelling the polysaccharide may be 70 to 100°C or 80 to 90°C, for example. The polysaccharide may be swelled while being stirred under conditions of 1000 to 5000 rpm, for example. The swelled polysaccharide is allowed to stand for cooling, and then to be pulverized, or the swelled polysaccharide is cooled while being stirred, to obtain a polysaccharide gel particle having a mean particle size of 0.1 to 1000 µm. The pulverization can be carried out using a homogenizer, Disper mixer or blender, for example. The pulverization may alternatively be carried out by stirring at 5000 rpm to 20,000 rpm. A gelling agent (such as calcium chloride or potassium chloride) may also be used during the process of producing the gel particles. When a gelling agent is used for gelation of the polysaccharide, it is preferred to select one such that the jelly strength (or gelation strength) of the polysaccharide is between 500 g/cm² and 1000 g/cm² at a polysaccharide concentration of 1.5 mass%, for example. The gel particles do not have a thickening effect that is effective enough to be used as a thickening agent for cosmetics.

When agar is used as a polysaccharide, as gel particles a pulverized form of an agar gel may be used. As an agar gel, agar swelled with the gel-forming aqueous phase of the second layer and gelated may be used. The pulverized form of an agar gel is obtained by allowing the swelled agar to stand for cooling then pulverizing the same or by cooling while stirring the swelled agar.

When the polysaccharide is at least one selected from the group consisting of carrageenan, gellan gum, sodium alginate, tamarind gum, mannan and locust bean gum, the gel particles may be a pulverized form of the polysaccharide gel, a polysaccharide gel obtained by gelating the polysaccharide swelled with the gel-forming aqueous phase of the second layer. In this case, gelation may be carried out by adding a gelling agent.

The mean particle size of the gel particles may be 0.1 µm or greater, 1 µm or greater, 5 µm or greater, 10 µm or greater, 50 µm or greater, 60 µm or greater, 70 µm or greater or 80 µm or greater, and no greater than 1000 µm, no greater than 500 µm, no greater than 400 µm, no greater than 300 µm, no greater than 250 µm, no greater than 200 µm, no greater than 150 µm or no greater than 120 µm. In other words, the mean particle size of the gel particle may be 0.1 to 1000 µm, 1 to 500 µm, 5 to 400 µm, 10 to 300 µm, 50 to 250 µm, 60 to 200 µm, 70 to 150 µm or 80 to 120 µm. The term "mean particle size" as used herein is defined as the value measured using a Laser scattering particle size distribution analyzer. The mean particle size of the gel particles can be adjusted to within such ranges by altering the conditions under which the polysaccharide gel particles are produced (such as the pulverizing conditions).

When the polysaccharide is agar, the jelly strength (or gelation strength) of the polysaccharide may be 500 g/cm² or greater, 600 g/cm² or greater, 650 g/cm² or greater or 700 g/cm² or greater, and no greater than 1000 g/cm², no greater than 900 g/cm², no greater than 800 g/cm² or no greater than 750 g/cm², with a polysaccharide concentration of 1.5 mass%. From the viewpoint of superior smoothness, when the polysaccharide is agar its jelly strength (or gelation strength) may be between 500 g/cm² and 1000 g/cm², between 600 g/cm² and 900 g/cm², between 650 g/cm² and 800 g/cm² or between 700 g/cm² and 800 g/cm², at a polysaccharide concentration of 1.5 mass%.

The jelly strength (or gelation strength) is measured as a gel strength for an aqueous solution having a 1.5% polysaccharide concentration In other words, for the jelly strength measurement, the polysaccharide is weighed accurately, and deionized water is added thereto, thereby causing the polysaccharide to sufficiently absorb water. Subsequently, warm deionized water is added thereto to adjust the content, which is then subjected to a hot water bath to cause dissolution by heat. In order to make up water evaporating by heating, deionized water is used as a supplement to adjust the content, and the solution is caused to flow into a glass container in which a tape is wound around the upper portion thereof. The container is left to cool at room temperature, and then capped and left in a constant temperature chamber at 20°C overnight. The tape is peeled off from the glass container, and then jelly around the periphery of the container that sticks out of the container is cut with a cutter and discarded. The strength of the cut surface of the obtained jelly is measured using a texture analyzer or the like. That is, a cylindrical plunger having an area of 1 cm² is mounted on the cut surface, and the sample stage is moved at an appropriate lifting rate. In this manner, a force applied until the jelly breaks can be measured.

From the viewpoint of allowing the gel particle to settle more easily, the weight-average molecular weight of the polysaccharide may be 150,000 or greater, 200,000 or greater, 250,000 or greater or 300,000 or greater, and from the viewpoint of superior smoothness (minimally rough surface), it is preferably no greater than 500,000, no greater than 450,000, no greater than 400,000 or no greater than 350,000. The weight-average molecular weight of the polysaccharide may be between 150,000 and 500,000, between 150,000 and 450,000, between 150,000 and 400,000, between 150,000 and 350,000, between 200,000 and 500,000, between 200,000 and 450,000, between 200,000 and 400,000, between 200,000 and 350,000, between 250,000 and 500,000, between 250,000 and 450,000, between 250,000 and 400,000, between 250,000 and 350,000, between 300,000 and 500,000, between 300,000 and 450,000, between 300,000 and 400,000 or between 300,000 and 350,000.

The weight-average molecular weight of the polysaccharide can be measured by HPLC gel permeation chromatography. For example, after dissolving the polysaccharide in distilled water at 95 to 97°C, the solution is cooled to 50°C to obtain a measuring sample, and the gel permeation chromatography measurement is conducted using this sample. One example of a liquid chromatography apparatus is LC-10AT VP or RID-10A by Shimadzu Corp., with a differential refractometer as the detector, TOSOH TSK-GEL for HPLC or TSK-GEL GMPWXL by Tosoh Corp. as the column and 0.1 M sodium nitrate as the developing solvent, and the measurement being conducted at a constant temperature. The weight-average molecular weight of agar is determined using pullulan of known molecular weight (Shodex STANDARD P-82, for example) as the standard sample. The standard sample is dissolved in distilled water, and measurement is performed by HPLC gel permeation chromatography under the same conditions.

The polysaccharide content may be 0.01 mass% or greater, 0.05 mass% or greater, 0.10 mass% or greater, 0.20 mass% or greater, 0.30 mass% or greater or 0.40 mass% or greater, and no greater than 2.0 mass%, no greater than 1.0 mass%, no greater than 0.8 mass% or no greater than 0.6 mass%, based on the total mass of the two-layer cosmetic. In other words, the polysaccharide content may be from 0.05 to 2.0 mass%, in particular from 0.10 to 1.0 mass%, or from 0.20 to 0.8 mass% or even from 0.40 to 0.6 mass%, based on the total mass of the two-layer cosmetic.

The polysaccharide gel particles content may be 5 mass% or greater, 10 mass% or greater or 15 mass% or greater, and no greater than 50 mass%, no greater than 30 mass% or no greater than 25 mass%, based on the total mass of the two-layer cosmetic. The polysaccharide gel particles content may be from 5 to 50 mass%, in particular from 10 to 30 mass% or even from 15 to 25 mass%, based on the total mass of the two-layer cosmetic.

The viscosity of the two-layer cosmetic may be no higher than 0,2 Pa.s, no higher than 0,1 Pa.s, no higher than 0,05 Pa.s or lower than 0,01 Pa.s, and it may be 0,001 Pa.s or higher, or 0,005 Pa.s or higher, for example, at 25°C. The viscosity of the two-layer cosmetic may be 0,001 Pa.s to 0,2 Pa.s, 0,001 Pa.s to 0,1 Pa.s, 0,001 Pa.s to 0,05 Pa.s, 0,001 Pa.s to less than 0,01 Pa.s, 0,005 Pa.s to 0,2 Pa.s, 0,005 Pa.s to 0,1 Pa.s, 0,005 Pa.s to 0,05 Pa.s or 0,005 Pa.s to less than 0,01 Pa.s, at 25°C.

The viscosity of the two-layer cosmetic may be measured based on the shear viscosity using a rotating viscometer (Rheolab QC by Anton Paar GmbH), under conditions of 100 rpm, 25°C. A viscosity in this range will improve the stability of the two-layer cosmetic and give it an excellent feel during use.

The redispersible two-layer cosmetic can be obtained, for example, by mixing (shaking) and stirring the aqueous phase of the first layer and gel particles of the second layer.

The redispersible two-layer cosmetic of this embodiment can be suitably used for a cosmetic water product, cleansing lotion, face cleanser, essence, makeup base, lotion mist, sunscreen or the like.
The present invention also relates to a cosmetic process for caring for and/or making-up keratinic materials comprising the application onto keratinic materials, in particular onto skin, of the redispersible two-layer cosmetic as defined in the invention.
By 'keratinic materials', it means skin and/or lips, preferably skin.
The redispersible two-layer cosmetic is generally shaken before use.
In particular, the redispersible two-layer cosmetic of the invention advantageously provides a long-lasting moisturizing effect and smoothness onto keratinic materials, in particular onto skin, on which it is applied.

### Examples

The invention will now be illustrated by examples, with the understanding that the invention is not meant to be limited to these examples. Unless contrary indication, the % are mass% also referred as % by weight of total weight of the composition.

The following polysaccharides were prepared.
· Agar 1 (trade name: Ina Agar CS-7, Ina Food Industry Co., Ltd., INCI name: AGAR, jelly strength (1.5 mass% concentration): 730 ±20 (g/cm³), weight-average molecular weight: 300,000).
· Agar 2 (trade name: Ina Agar CS-310, Ina Food Industry Co., Ltd., INCI name: AGAR, jelly strength (1.5 mass% concentration): 100 ±50 (g/cm³), weight-average molecular weight: 100,000).
· Agar 3 (trade name: Ina Agar CS-33, Ina Food Industry Co., Ltd., INCI name: AGAR, jelly strength (1.5 mass% concentration): 850 ±50 (g/cm³), weight-average molecular weight: 700,000).
· Carrageenan (trade name: GENUGEL^{R}, SWG-J type: kappa, CPKelco Co., Ltd., INCI name: CARRAGEENAN (Kappa)).
· Gellan gum (trade name: KELCOGEL, CPKelco Co., Ltd., INCI name: GELLAN GUM (LA type)).

The jelly strength of agar and the weight-average molecular weight are the values measured by the above-mentioned method.

Polysaccharide gel particles were prepared from the polysaccharide by the following method. First, water (the gel-forming aqueous phase of the second layer) and the polysaccharide were combined, and while the combination was stirred at 90°C, 3000 rpm, the polysaccharide was swelled. When the agar was used as the polysaccharide, the swelled polysaccharide was allowed to stand for cooling to obtain an agar gel (polysaccharide concentration: 4 mass%). As a gelling agent, potassium chloride was added to the kappa carrageenan and mixed to obtain a kappa carrageenan gel (polysaccharide concentration: 4 mass%). As a gelling agent, calcium chloride was added to the gellan gum and mixed to obtain a gellan gum gel (polysaccharide concentration: 4 mass%). Water (the gel-forming aqueous phase of the second layer) was added to the gel which was then pulverized with a Waring blender at 18,000 rpm for 2 minutes to prepare polysaccharide gel particles (polysaccharide concentration: 2 mass%).

In Example 1 and Examples 4 to 9, the mean particle size of the agar gel particles was 100 µm, in Example 2 the mean particle size of the carrageenan gel particles was 100 µm, and in Example 3 the mean particle size of the gellan gum gel particles was 100 µm. The mean particle size of the gel particles was measured using a Laser scattering particle size distribution analyzer.

Aqueous phases (first layer) having the compositions listed in Tables 1 and 2 were added to the polysaccharide gel particles obtained by the method described above, and the mixtures were stirred to prepare two-layer cosmetics for the Examples. The polysaccharide contents of the two-layer cosmetics of the Examples were the amounts listed in Table 1 with respect to the total masses of the two-layer cosmetics.

Agar (trade name: Ina Agar CS-7, Ina Food Industry Co., Ltd.) and water were combined, and while the combination was stirred at 90°C, 3000 rpm, the ager was swelled. An aqueous phase (first layer) having the composition listed in Table 1 was added to the swelled agar and the mixture was stirred to prepare a cosmetic for Comparative Example 1 and 2. Bentonite and kaolin (powder second phase) were also mixed with an aqueous phase (first phase) having the composition listed in Table 1 to prepare a two-layer cosmetic for Comparative Example 3. Oil (second phase) having the composition listed in Table 1 were mixed with an aqueous phase (first phase) having the composition listed in Table 1 to prepare a two-layer cosmetic for Comparative Example 4. A cosmetic having the composition listed in Table 2 was also prepared as a cosmetic for Comparative Example 5.

The viscosities of the two-layer cosmetics were measured by the following method. The shear viscosity of each cosmetic was measured at 25°C using a rotating viscometer (Rheolab QC by Anton Paar GmbH) (rotational speed: 100 rpm).

The cosmetics of the Examples and Comparative Examples were evaluated for "separation", "re-dispersibility", "long-lasting moisturizing effect" and "smoothness", on the following scales. The "long-lasting moisturizing effect" and "smoothness" parameters were evaluated by a single use test on skin by an evaluation panel of cosmetic experts.
(1) Separation After mixing by shaking followed by standing at room temperature, the outer appearance was visually evaluated.
   A: The interface between a uniform upper layer and a uniform lower layer was clearly observable after standing for half a day
   B: The interface between a uniform upper layer and a uniform lower layer was clearly observable after standing for a full day
   C: The interface between the upper layer and lower layer was observable but indistinct after shaking and standing for a full day, with non-uniformity of either the upper layer or lower layer
   D: No observable interface between the upper layer and lower layer after shaking and standing for a full day
(2) Re-dispersibility The mixture was shaken and allowed to stand for 1 month at 25°C, and then shaken again.
   A: Uniformly dispersed after shaking ≤10 times
   B: Uniformly dispersed after shaking 10 to ≤20 times
   C: Dispersed but non-uniform after ≥20 times
   D: No re-dispersion
(3) Long-lasting moisturizing effect
   A: Very notable long-lasting moisturizing effect
   B: Notable long-lasting moisturizing effect
   C: Virtually no notable long-lasting moisturizing effect
   D: Absolutely no notable long-lasting moisturizing effect
(4) Smoothness
   A: Very notable smoothness
   B: Notable smoothness
   C: Virtually no notable smoothness
   D: Absolutely no notable smoothness

**[Table 1]**

| | | Example | | | Comp. Example | | | |
|---|---|---|---|---|---|---|---|---|
| | | 1 | 2 | 3 | 1 | 2 | 3 | 4 |
| polysaccharide, aqueous phase (invention), or powder phase (comparative) | Agar (mass%) | 0.5 | - | - | 0.5 | 0.2 | - | - |
| | Carrageenan (mass%) | - | 0.5 | - | - | - | - | - |
| | Gellan gum (mass%) | - | - | 0.5 | - | - | - | - |
| | Potassium chloride (mass%) | - | 1 | - | - | - | - | - |
| | Calcium chloride (mass%) | - | - | 1 | - | - | - | - |
| | Bentonite (mass%) | - | - | - | - | - | 0.5 | - |
| | Kaolin (mass%) | - | - | - | - | - | 0.5 | - |
| | Magnesium sulfate (mass%) | - | - | - | - | - | 1 | - |
| | Butylene glycol (mass%) | 5 | 5 | 5 | 5 | 5 | 5 | 5 |
| | Glycerin (mass%) | 5 | 5 | 5 | 5 | 5 | 5 | 5 |
| | Phenoxyethanol (preservative) (mass%) | 1 | 1 | 1 | 1 | 1 | 1 | 1 |
| | Ethanol (mass%) | 5 | 5 | 5 | 5 | 5 | 5 | 5 |
| | PPG-6 decyl tetradeceth-20 (NIKKOL PEN-4620, solubilizer) (mass%) | 0.5 | 0.5 | 0.5 | 0.5 | 0.5 | 0.5 | - |
| | Water | rem. | rem. | rem. | rem. | rem. | rem. | rem. |
| Oil phase (comparative) | Triethylhexannin (mass%) | - | - | - | - | - | - | 5 |
| | Squalane (mass%) | - | - | - | - | - | - | 5 |
| Perfume (mass%) | | 0.1 | 0.1 | 0.1 | 0.1 | 0.1 | 0.1 | 0.1 |
| Total amount (mass%) | | 100 | 100 | 100 | 100 | 100 | 100 | 100 |
| Evaluation | Viscosity (Pa.s) | <0,01 | <0,0 1 | <0,0 1 | >0,2 | >0,05 | <0,01 | <0,01 |
| | Separation | A | B | B | D | C | B | B |
| | Redispersibility | A | A | A | D | C | B | B |
| | Long-lasting moisturizing effect | A | A | A | B | B | D | C |
| | Smoothness | A | B | B | C | C | B | D |

| | | | | | | | | |
|---|---|---|---|---|---|---|---|---|
| The term'rem'. means 'qs100'. | | | | | | | | |

**[Table 2]**

| | | | Comp. Example | Example | | | | | |
|---|---|---|---|---|---|---|---|---|---|
| | | | 5 | 4 | 5 | 6 | 7 | 8 | 9 |
| polysaccharide, aqueous phase, powder | Agar (mass%) | CS-7 | - | 0.2 | 0.4 | 0.6 | - | - | - |
| | | YAWARA | - | - | - | - | 0.5 | - | - |
| | | CS-310 | - | - | - | - | - | 0.5 | - |
| | | CS-33 | - | - | - | - | - | - | 0.5 |
| | Butylene glycol (mass%) | | 5 | 5 | 5 | 5 | 5 | 5 | 5 |
| | Glycerin (mass%) | | 5 | 5 | 5 | 5 | 5 | 5 | 5 |
| | Phenoxyethanol (preservative) (mass%) | | 1 | 1 | 1 | 1 | 1 | 1 | 1 |
| | Ethanol (mass%) | | 5 | 5 | 5 | 5 | 5 | 5 | 5 |
| | Perfume (mass%) | | 0.1 | 0.1 | 0.1 | 0.1 | 0.1 | 0.1 | 0.1 |
| | PPG-6 decyl tetradeceth-20 (NIKKOL PEN-4620, solubilizer) (mass%) | | 0.5 | 0.5 | 0.5 | 0.5 | 0.5 | 0.5 | 0.5 |
| | Water | | rem. | rem. | rem. | rem. | rem. | rem. | rem. |
| | Total amount (mass%) | | 100 | 100 | 100 | 100 | 100 | 100 | 100 |
| Evaluation | Viscosity (Pa.s) | | <0,01 | <0,01 | <0,01 | <0,01 | <0,01 | <0,01 | <0,01 |
| | Separation | | D | A | B | B | B | B | A |
| | Redispersibility | | D | A | A | A | A | A | A |
| | Long-lasting moisturizing effect | | D | A | A | A | A | B | A |
| | Smoothness | | D | A | B | B | B | A | B |

These results showed that the redispersible two-layer cosmetic comprising an aqueous phase and a polysaccharide gel particles having a mean particle size of 0.1 to 1000 µm according to the invention are well redispersible and have improved long-lasting moisturizing effect and smoothness in comparison to other two-layer cosmetic known from prior art.

## Claims

1. A redispersible two-layer cosmetic comprising an aqueous phase and polysaccharide gel particles having a mean particle size of 0.1 to 1000 µm, wherein the aqueous phase comprises a polyol and/or a mono-alcohol, and wherein the redispersible two-layer cosmetic consists of a bottom layer formed by a plurality of gel particles aggregates, and an upper layer composed of the aqueous phase over the bottom layer, wherein the mean particle size is measured using a Laser scattering particle size distribution analyzer.

2. The redispersibe two-layer cosmetic according to claim 1, wherein the polysaccharide is selected from the group consisting of agar, carrageenan, gellan gum, sodium alginate, tamarind gum, mannan, locust bean gum, and mixtures thereof, in particular agar, carrageenan and gellan gum, and more preferably agar.

3. The redispersibe two-layer cosmetic according to any one of claim 1 or 2, wherein the viscosity of the two-layer cosmetic is 0,001 Pa.s to 0,05 Pa.s at 25°C, wherein the viscosity is measured based on the shear viscosity using a rotating viscometer under conditions of 100 rpm, 25°C.

4. The redispersible two-layer cosmetic according to any one of claims 1 to 3, wherein the polyol is selected from the group consisting of diols, triols and mixtures thereof, in particular butylene glycol (1,3-butylene glycol), pentylene glycol, propanediol, dipropylene glycol, polyalkylene glycols, glycerin, and mixtures thereof.

5. The redispersible two-layer cosmetic according to any one of claims 1 to 4, wherein the mono-alcohol is selected from the group consisting of ethanol, propanol, isopropanol and butanol, preferably ethanol.

6. The redispersible two-layer cosmetic according to any one of claims 1 to 5, wherein the aqueous phase comprises an aryloxyalkanol, preferably phenoxyethanol.

7. The redispersible two-layer cosmetic according to any one of claims 1 to 6, which is water product, a cleansing lotion, a face cleanser, an essence, a makeup base, a lotion mist, or a sunscreen.

8. Cosmetic process for caring for and/or making-up keratinic materials comprising the application onto keratinic materials, in particular onto skin, of the redispersible two-layer cosmetic as defined in any one of the claims 1 to 7.

9. Cosmetic process according to claim 8, wherein the redispersible two-layer cosmetic is shaken before use.

10. Cosmetic process according to claim 8 or 9, wherein said two-layer cosmetic provides a long-lasting moisturizing effect and smoothness onto keratinic materials, in particular onto skin, on which it is applied.

## Patentansprüche

1. Redispergierbares, aus zwei Schichten bestehendes kosmetisches Produkt, das eine wässrige Phase und Polysaccharidgelpartikel umfasst, die eine mittlere Partikelgröße von 0,1 bis 1000 µm aufweisen, wobei die wässrige Phase ein Polyol und/oder einen Monoalkohol umfasst und wobei das redispergierbare, aus zwei Schichten bestehende kosmetische Produkt aus einer unteren Schicht, die von einer Vielzahl von Gelpartikelaggregaten gebildet ist, und einer oberen Schicht besteht, die aus der wässrigen Phase über der unteren Schicht besteht, wobei die mittlere Partikelgröße unter Verwendung eines Laserstreuungs-Partikelgrößenverteilungsanalysators gemessen wird.

2. Redispergierbares, aus zwei Schichten bestehendes kosmetisches Produkt nach Anspruch 1, wobei das Polysaccharid ausgewählt ist aus der Gruppe, die besteht aus Agar, Carrageen, Gellangummi, Natriumalginat, Tamarindengummi, Mannan, Johannisbrotgummi und Gemischen davon, insbesondere Agar, Carrageen und Gellangummi und bevorzugter Agar.

3. Redispergierbares, aus zwei Schichten bestehendes kosmetisches Produkt nach einem der Ansprüche 1 oder 2, wobei die Viskosität des aus zwei Schichten bestehenden kosmetischen Produkts bei 25°C 0,001 Pa.s bis 0,05 Pa.s beträgt, wobei die Viskosität basierend auf der Scherviskosität unter Verwendung eines Rotationsviskosimeters unter Bedingungen von 100 U/min, 25°C gemessen wird.

4. Redispergierbares, aus zwei Schichten bestehendes kosmetisches Produkt nach einem der Ansprüche 1 bis 3, wobei das Polyol ausgewählt ist aus der Gruppe, die besteht aus Diolen, Triolen und Gemischen davon, insbesondere Butylenglycol (1,3-Butylenglycol), Pentylenglycol, Propandiol, Diproylenglycol, Polyalkylenglycolen, Glycerin und Gemischen davon.

5. Redispergierbares, aus zwei Schichten bestehendes kosmetisches Produkt nach einem der Ansprüche 1 bis 4, wobei der Monoalkohol ausgewählt ist aus der Gruppe, die besteht aus Ethanol, Propanol, Isopropanol und Butanol, vorzugsweise Ethanol.

6. Redispergierbares, aus zwei Schichten bestehendes kosmetisches Produkt nach einem der Ansprüche 1 bis 5, wobei die wässrige Phase einen Aryloxyalkanol, vorzugsweise Phenoxyethanol, umfasst.

7. Redispergierbares, aus zwei Schichten bestehendes kosmetisches Produkt nach einem der Ansprüche 1 bis 6, das ein Wasserprodukt, eine Reinigungslotion, ein Gesichtsreinigungsmittel, eine Essenz, eine Makeup-Basis, ein Lotionsnebel oder eine Sonnencreme ist.

8. Kosmetisches Verfahren zur Pflege und/oder zum Make-up keratinischer Materialien, welches das Aufbringen des redispergierbaren, aus zwei Schichten bestehenden kosmetischen Produkts nach einem der Ansprüche 1 bis 7 auf keratinische Materialien, insbesondere auf Haut, umfasst.

9. Kosmetisches Verfahren nach Anspruch 8, wobei das redispergierbare, aus zwei Schichten bestehende kosmetische Produkt vor der Verwendung geschüttelt wird.

10. Kosmetisches Verfahren nach Anspruch 8 oder 9, wobei das aus zwei Schichten bestehende kosmetische Produkt eine langanhaltende feuchtigkeitsspendende Wirkung und Glätte auf keratinischen Materialien, insbesondere auf Haut, auf denen sie aufgetragen wird, bereitstellt.

## Revendications

1. Cosmétique bicouche redispersible comprenant une phase aqueuse et des particules de gel de polysaccharide ayant une taille moyenne de 0,1 à 1000 µm, dans lequel la phase aqueuse comprend un polyol et/ou un mono-alcool, le cosmétique bicouche redispersible consistant en une couche inférieure formée par une pluralité d'agrégats de particules de gel, et une couche supérieure composée de la phase aqueuse sur la couche inférieure, la taille moyenne des particules étant mesurée à l'aide d'un analyseur de granulométrie par diffraction laser.

2. Cosmétique bicouche redispersible selon la revendication 1, dans lequel le polysaccharide est choisi dans le groupe constitué par l'agar, la carraghénane, la gomme gellane, l'alginate de sodium, la gomme de tamarin, la mannane, la gomme de caroube, et leurs mélanges, en particulier l'agar, la carraghénane et la gomme gellane, et plus préférentiellement l'agar.

3. Cosmétique bicouche redispersible selon l'une des revendications 1 ou 2, dans lequel la viscosité du cosmétique bicouche est comprise entre 0,001 Pa.s et 0,05 Pa.s à 25°C, la viscosité étant mesurée sur la base de la viscosité de cisaillement à l'aide d'un viscosimètre rotatif dans des conditions de 100 rpm, 25°C.

4. Cosmétique bicouche redispersible selon l'une quelconque des revendications 1 à 3, dans lequel le polyol est choisi dans le groupe constitué par les diols, les triols et leurs mélanges, en particulier le butylène glycol (1,3-butylène glycol), le pentylène glycol, le propanediol, le dipropylène glycol, les polyalkylène glycols, la glycérine et leurs mélanges.

5. Cosmétique bicouche redispersible selon l'une quelconque des revendications 1 à 4, dans lequel le mono-alcool est choisi dans le groupe constitué par l'éthanol, le propanol, l'isopropanol et le butanol, de préférence l'éthanol.

6. Cosmétique bicouche redispersible selon l'une quelconque des revendications 1 à 5, dans lequel la phase aqueuse comprend un aryloxyalcanol, de préférence le phénoxyéthanol.

7. Le cosmétique bicouche redispersible selon l'une des revendications 1 à 6, qui est un produit à base d'eau, une lotion nettoyante, un nettoyant pour le visage, une essence, une base de maquillage, une brume lotion ou un écran solaire.

8. Procédé cosmétique de soin et/ou de maquillage des matières kératiniques comprenant l'application sur les matières kératiniques, notamment sur la peau, du cosmétique bicouche redispersible tel que défini dans l'une quelconque des revendications 1 à 7.

9. Procédé cosmétique selon la revendication 8, dans lequel le cosmétique bicouche redispersible est agité avant utilisation.

10. Procédé cosmétique selon la revendication 8 ou 9, dans lequel ledit cosmétique bicouche procure un effet hydratant et doux de longue durée sur les matières kératiniques, en particulier sur la peau, sur laquelle il est appliqué.
